(19) Europäisches Patentamt
European Patent Office
Office européen des brevets

(11) Veröffentlichungsnummer: **0 027 956**
**A1**

(12) # EUROPÄISCHE PATENTANMELDUNG

(21) Anmeldenummer: **80106254.8**

(22) Anmeldetag: **15.10.80**

(51) Int. Cl.³: **C 07 D 209/28**

(30) Priorität: **25.10.79 DE 2943125**

(43) Veröffentlichungstag der Anmeldung: **06.05.81 Patentblatt 81/18**

(84) Benannte Vertragsstaaten: **AT BE CH DE FR GB IT LI LU NL SE**

(71) Anmelder: **Troponwerke GmbH & Co. KG, Berliner Strasse 156, D-5000 Köln 80 (DE)**

(72) Erfinder: **Boltze, Karl-Heinz, Dr., Am Burgtor 23, D-5060 Bergisch-Gladbach (DE)**
Erfinder: **Raddatz, Siegfried, Dr., Jakob-Boehme-Strasse 21, D-5000 Köln 80 (DE)**
Erfinder: **Seidel, Peter-Rudolf, Dr., Alte Heide 50, D-5000 Köln 90 (DE)**

(74) Vertreter: **Dill, Erwin, Dr. et al, c/o BAYER AG Zentralbereich Patente Marken und Lizenzen Bayerwerk, D-5090 Leverkusen 1 (DE)**

(54) **Verfahren zur Herstellung von (1-(4-Chlorbenzoyl)-5-methoxy-2-methyl-3-indol)acetoxyessigsäure.**

(57) Die vorliegende Anmeldung betrifft ein Verfahren zur Herstellung der bekannten [1-(4-Chlorobenzoyl)-5-methoxy-2-methyl-3-indol]acetessigsäure von besonderer Reinheit.

Die nach dem beschriebenen Verfahren hergestellte Endverbindung ist ein wertvoller pharmazeutischer Wirkstoff mit antiphlogistischer Wirkung.

EP 0 027 956 A1

Troponwerke GmbH & Co. KG

KS/bc/c
24. Okt. 1979

Verfahren zur Herstellung von [1-(4-Chlorbenzoyl)-5-methoxy-2-methyl-3-indol]acetoxyessigsäure

Die vorliegende Erfindung betrifft ein neues, chemisch eigenartiges und vorteilhaftes Verfahren zur Herstellung der bekannten [1-(4-Chlorbenzoyl)-5-methoxy-2-methyl-3-indol]acetessigsäure von besonderer Reinheit.

Für die Herstellung dieser bekannten Verbindung sind bereits eine Reihe von Verfahren bekannt geworden (vgl. DT-OS 2 234 651 und DT-OS 2 257 867).

Bei all diesen Verfahren wurde die Carboxylgruppe zunächst durch einen Benzylrest geschützt, so daß in einem letzten Reaktionsschritt eine katalytische Hydrierung des Benzylesters gemäß folgendem Reaktionsschema durchgeführt werden mußte.

Reaktionsschema:

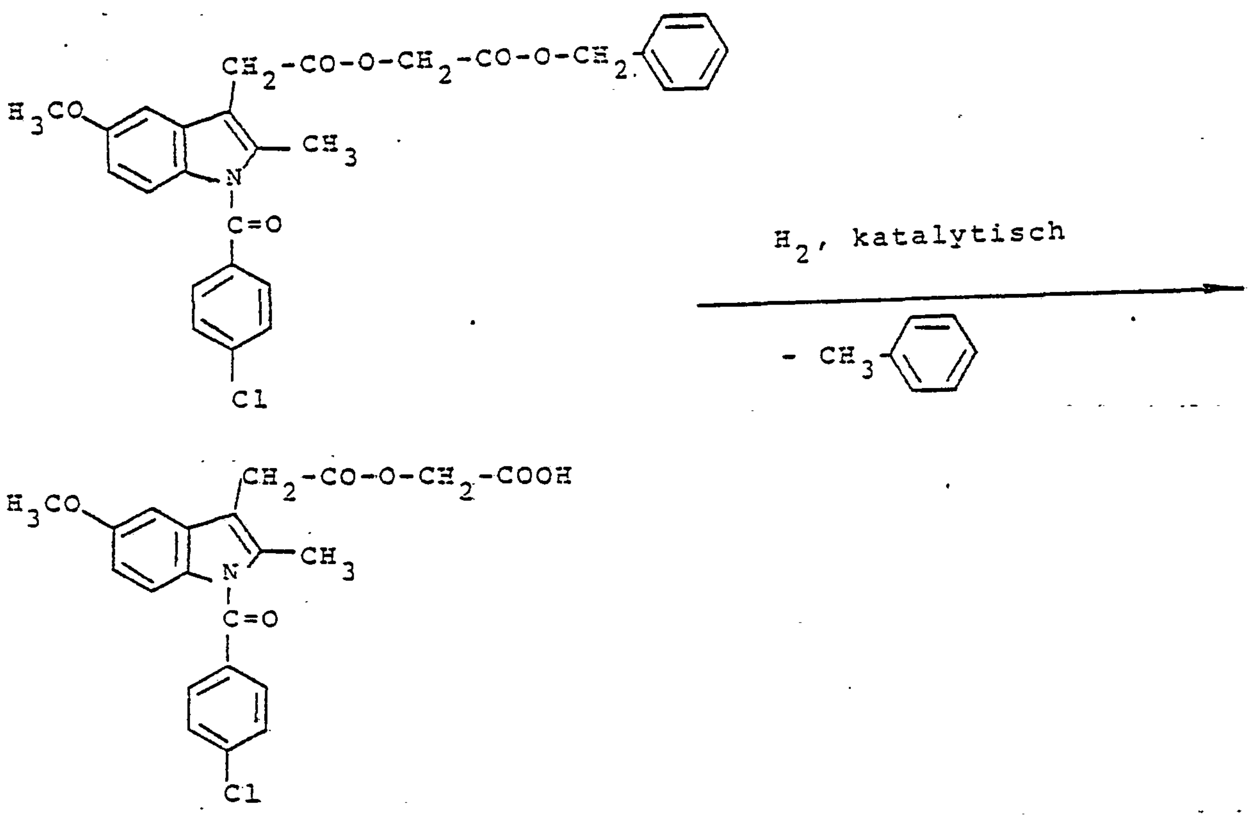


Bei dieser Abspaltung des Benzylrestes entsteht als Nebenprodukt stets die (1-Benzoyl-5-methoxy-2-methyl-3-indol)acetoxyessigsäure (Des-Chlor-Verbindung). Diese unerwünschte Verunreinigung, die durch Abspaltung des Chlors aus dem Benzolring des 4-Chlorbenzoylrestes bis zu einer Menge von 0,5 % entsteht, muß dann in aufwendigen Reinigungsschritten anschließend entfernt werden, was mit Ausbeuteverlusten verbunden ist.

Aufgabe der vorliegenden Erfindung ist es, ein alternatives Herstellungsverfahren zur Verfügung zu stellen, bei welchem die unerwünschte Des-Chlor-Verbindung nicht entsteht.

Überraschenderweise wurde gefunden, daß man die [1-(4-Chlorbenzoyl)-5-methoxy-2-methyl-3-indol]acetoxyessigsäure in einfacher Weise und großer Reinheit erhält, wenn man α-(4-Chlorbenzoyl)-4-methoxyphenylhydrazin-hydrochlorid der Formel (I)

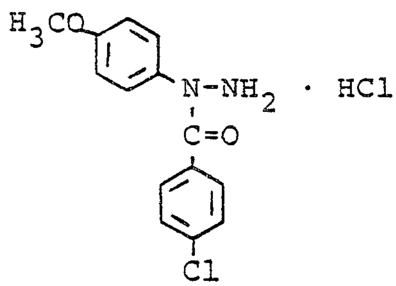

(I)

oder das entsprechende Sulfonat,
mit der freien Lävulinoyloxyessigsäure der Formel (II)

$$CH_3-CO-CH_2-CH_2-COO-CH_2-COOH \qquad (II)$$

in Eisessig als Lösungsmittel, bei Temperaturen zwischen 20 und 60°C umsetzt und das hierbei zunächst entstehende Hydrazon der Formel (III)

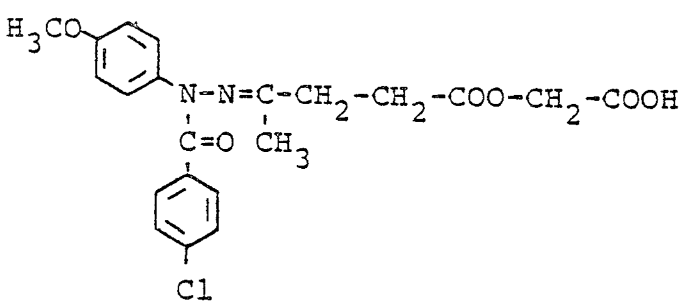

(III)

durch Erhitzen auf 40 bis 80°C, unter Ammoniakabspaltung zum gewünschten Indol cyclisiert.

Es konnte nicht erwartet werden, daß nach diesem Verfahren die [1-(4-Chlorbenzoyl)-5-methoxy-2-methyl-3-indol]acetoxyessigsäure in so reiner Form, d.h. frei von der störenden Des-Chlor-Verbindung, und in Ausbeuten von 60 bis 70 % der Theorie entsteht.

Die bei der Durchführung des erfindungsgemäßen Verfahrens eingesetzte Hydrazinverbindung der Formel (I) ist bekannt (vgl. britisches Patent 1 148 909).

Die Lävulinoyloxyessigsäure der Formel (II) ist bisher noch nicht bekannt, kann aber in einfacher Weise hergestellt werden, indem man den bekannten Lävolinoyloxyessigsäurebenzylester (Lit.: Tatsuo Yamanaka u. Heinosuke Yasuda, Scientific Research Institute, Ltd., Japan 272 ('58), Jan. 23 und DT-OS 2 234 651) katalytisch hydriert.

Die nach dem erfindungsgemäßen Verfahren hergestellte Endverbindung ist ein wertvoller pharmazeutischer Wirkstoff mit antiphlogistischer Wirkung (vgl. DT-PS 2 234 651).

## Ausführungsbeispiele

### Beispiel 1

0,8 bis 0,6 ml Lävulinoyloxyessigsäure werden in 800 bis 600 ml Eisessig gelöst und bei 50°C mit 0,4 Mol α-(4-Chlorbenzoyl)-4-methoxyphenylhydrazinhydrochlorid versetzt. Man läßt 2 Stunden unter Stickstoffatomosphäre bei 50 bis 70°C reagieren. Beim Abkühlen und nach Animpfen mit einigen Kristallen der [1-(4-Chlorbenzoyl)-5-methoxy-2-methyl-3-indol]acetoxyessigsäure fällt das Produkt in gelben Kristallen aus, Fp: 151 bis 152°C.
Ausbeute: 65 % der Theorie.

### Beispiel 2

0,36 Mol Lävulinoyloxyessigsäure werden in 350 ml Eisessig gelöst und bei 50°C mit 0,24 Mol fein gemahlenem α-(4-Chlorbenzoyl)-4-methoxyphenylhydrazinhydrochlorid versetzt.

Unter Stickstoff läßt man 1 Stunde bei 55 bis 57°C rühren, erhöht dann die Temperatur um 5°C und läßt noch eine weitere Stunde reagieren. Man gibt zu dem heißen Ansatz 265 ml Wasser und startet die Kristallisation durch Animpfen mit einigen Kristallen. Unter Rühren versetzt man innerhalb von 30 Minuten mit weiteren 175 ml Wasser, bis 20°C erreicht sind. Man rührt noch 60 Minuten nach und filtriert dann

das ausgefallene Produkt ab. Es wird mit 100 ml 30 %iger Essigsäure und 500 ml Wasser gewaschen und ergibt ein farbloses, kristallines Produkt. Die Rohausbeute dieses wasserhaltigen Produktes beträgt 90 % der Theorie. Der Wassergehalt wird zu 20 % bestimmt, so daß in diesem Rohprodukt 70 % der Theorie des Endproduktes enthalten sind. Dieses wird 1 Stunde bei 90°C unter Wasserstrahlvakuum getrocknet. Man erhält gelbliche Kristalle, die bei 151 bis 152°C schmelzen.
Ausbeute: 63 % der Theorie.

Patentansprüche

1) Verfahren zur Herstellung von (1-(4-Chlorbenzoyl)-5-methoxy-2-methyl-3-indol)acetoxyessigsäure, dadurch gekennzeichnet, daß man α-(4-Chlorbenzoyl)-4-methoxyphenylhydrazin-hydrochlorid der Formel (I)

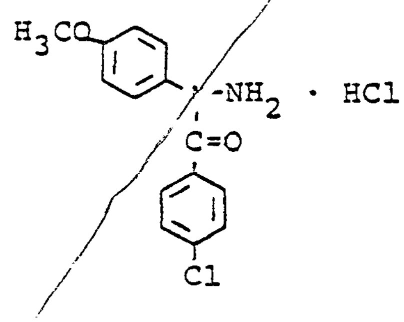

(I)

oder das entsprechende Sulfonat mit der freien Lävulinoyloxyessigsäure der Formel (II)

$$CH_3-CO-CH_2-CH_2-COO-CH_2-COOH \qquad (II)$$

in Eisessig als Lösungsmittel bei Temperaturen zwischen 20 und 60°C umsetzt und das hierbei zunächst entstehende Hydrazon der Formel (III)

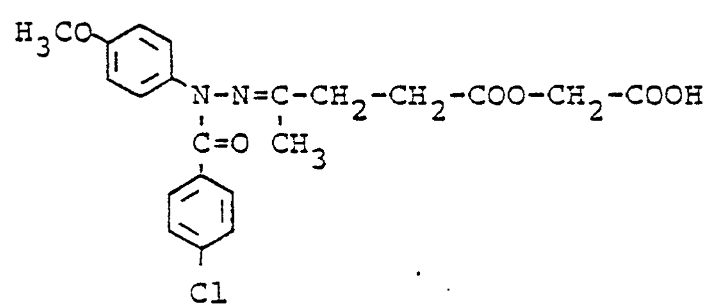

(III)

durch Erhitzen auf 40 bis 80°C, gegebenenfalls unter Inertgasatmosphäre, unter Ammoniakabspaltung zum gewünschten Indol cyclisiert.

2) Verfahren gemäß Anspruch 1, dadurch gekennzeichnet, daß die Cyclisierung zum Indol unter Inertgas-Atmosphäre durchgeführt wird.

0027956

Nummer der Anmeldung

EP 80 10 6254.8

Europäisches Patentamt

## EUROPÄISCHER RECHERCHENBERICHT

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| X | DE -A1 - 2 740 852 (TROPONWERKE GMBH & CO.)<br>* Seite 16 unten *<br>-- | 1,2 | C 07 D 209/28 |
| X | DE - A1 - 2 740 836 (TROPONWERKE GMBH & CO.)<br>* Beispiel 2 *<br>-- | 1 | |
| X | DE - A1 - 2 752 361 (TROPONWERKE GMBH & CO.)<br>* Seite 23 oben *<br>-- | 1 | RECHERCHIERTE SACHGEBIETE (Int. Cl.3) |
| | DE - A1 - 2 727 829 (HISAMITSU PHARMACEUTICAL CO.)<br>* Schema Seite 7 *<br>-- | 1 | C 07 D 209/28 |
| | US - A - 3 161 654 (MERCK & CO.)<br>* Spalte 4, Zeilen 44 bis 45 *<br>---- | 1 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 30-01-1981 | FROELICH |